# EUROPEAN PATENT SPECIFICATION

(11) **EP 2 931 328 B1**
(45) Date of publication and mention of the grant of the patent: **21.02.2018**
(21) Application number: 12823176.8
(22) Date of filing: 19.12.2012
(51) Int. Cl.: A61L 29/08, C08L 39/06

(54) **A READY FOR USE HYDROPHILIC CATHETER SET THAT DOES NOT LOSE ITS LUBRICIOUS EFFICIENCY WHEN LEFT IN WATER FOR A EXTENDED PERIOD OF TIME, WITH INCREASED LUBRICIOUS CHARACTERISTICS, AND A PRODUCTION METHOD FOR THIS CATHETER SET**
GEBRAUCHSFERTIGES HYDROPHILES KATHETERSET OHNE VERLUST DER GLEITFÄHIGKEIT BEI LANGZEITLAGERUNG IN WASSER UND MIT VERBESSERTEN GLEITEIGENSCHAFTEN SOWIE HERSTELLUNGSVERFAHREN FÜR DIESES KATHETERSET
JEU DE CATHÉTERS HYDROPHILES PRÊTS À L'EMPLOI DONT L'EFFICACITÉ LUBRIFIANTE RESTE INTACTE APRÈS IMMERSION DANS L'EAU PENDANT UNE LONGUE DURÉE, AUX CARACTÉRISTIQUES LUBRIFIANTES AMÉLIORÉES, ET MÉTHODE DE PRODUCTION DE CE JEU DE CATHÉTERS

(30) Priority: 13.12.2012 TR 201214643
(43) Date of publication of application: 21.10.2015
(73) Proprietor: Istem Medikal Tibbi Cihaz ve Sanayi Limited Sirketi, 06909 Maliköy Sincan, Ankara (TR)
(72) Inventor: DEMIREL, Miyaser, 06378 Ankara (TR)
(74) Representative: Berkkam, Ayfer
(86) International application number: PCT/TR2012/000224
(87) International publication number: WO 2014/092660

(56) References cited:
- WO-A1-98/11932
- US-A1- 2004 127 598
- US-A1- 2005 220 843

## Description

### Technical Field

This invention is related to a ready for use hydrophilic catheter set that does not lose its lubricious efficiency when left in water for an extended period of time, with increased lubricious characteristics, and a production method for this catheter set.

### Prior Art

In temporary catheterization basically a catheter with a hydrophilic coated surface is used. This catheter with a hydrophilic coated surface is located within a package. When the catheter is to be used the hydrophilic surface must be brought into contact with water or an aqueous solution for it to gain its lubricious characteristics. This results in the appearance of the problem of water or a solution to be carried around or somehow procured to be poured into the packaging for the catheter to be used.

Various applications for the solution of this problem are known. For example in European patents EP 1647298 and EP 0923398 refers to a catheter set comprising at least one catheter having on at least a part of its surface a hydrophilic surface layer intended to produce a low-friction surface character of the catheter by treatment with a liquid swelling medium prior to use of the catheter and a catheter package having a cavity for accommodation of the catheter and a closed catheter package manufactured from liquid sealed film material, characterized in that the package includes a compartment having walls of a gas impermeable material, said compartment accommodating said liquid swelling medium for provision of a ready-to-use catheter assembly, said compartment bag shaped or ampoule shaped within the cavity, said compartment shall be set so that liquid flow communication shall not be made until the catheter preparation is done before use from the cavity.

In this embodiment water or solution to come into contact with the hydrophilic surface in order for the lubricious characteristics to be present are stored in a separate compartment inside the catheter package. When the catheter is to be used the water or solution in a separate compartment is sent to the cavity housing the catheter and the catheter's hydrophilic surface is activated.

However some problems are encountered in this embodiment. First of all in order for the catheter set to be ready to use the water or solution within the compartment must be delivered to the cavity housing the catheter. This requires manual skill from the user. Taking into account that most of the users have paraplegia a solution for this problem is required.

Another issue encountered in this embodiment is time. After the water or solution is directed to the cavity housing the catheter, a waiting period is required for the activation of the hydrophilic surface. Therefore the catheter set is not ready for use and the directing of the water and activation of the hydrophilic surface must be completed before use.

Another issue with this embodiment is that the hydrophilic surface is not activated with water at the desired homogeneity. After the water is directed to the cavity housing the catheter, the water coming into contact with the whole of the hydrophilic surface is attempted by movement of the set (preferably by shaking). However, contact of the water to every point of the surface at the same homogeneity is not guaranteed and cases where the hydrophilic surface is not adequately moisturized and therefore the shaking process takes a long time and the lubricant is not at the desirable level and the quality of the hydrophilic surface is adversely affected are encountered.

Various enhancements have been made to resolve these issues; work has been carried out to place the catheter with a hydrophilic surface directly within the solution. The catheter set placed directly in the solution or water resolves the above mentioned issues. However placing the hydrophilic surface in water or solution for an extended period in time causes the hydrophilic surface to lose its lubrication and separate from the surface of the catheter.

For example, the patent document numbered TR 200909569 describes an embodiment where PVP (Polyvinylpyrrolidone) is used as the coating material for hydrophilicity and lubrication enhancer, methylene chloride is used as a solvent, Erucamide is used as a lubrication and stability enhancer, Toluene diisocyanate (TDI) is used as a cross linker, Isopropyl alcohol is used as a surface cleaner, sodium chloride is used as a glidant, Chitosan is used as an antibacterial agent and lubrication enhancer, Polyvinyl alcohol (PVA) is used as a lubrication enhancer, Acrylic acid is used as a solvent, Toluene and Benzoyl peroxide is used as a surface cleaner and distilled water is used as a solvent.

One of the issues encountered is that when the catheter with a hydrophilic surface is left the solution for an extended duration, in time the lubricious characteristic is lost. Another factor is that recently PVC-free materials are preferred as a support material to the catheter to be used. Currently catheters are manufactured from PVC materials of medical purity. However, especially in the light of European Union Directives, various limitation on the use of PVC in plastic materials are imposed, thus there is an inclination to reduce the use of PVC in plastic materials. In this context, the desired outcome is the hydrophilic surface's adherence to the catheters made from PVC-free materials and preservation of their lubricious characteristics for an extended period of time.

For this purpose, the need has arose for a catheter set that does not require an extra water filled compartment and is ready for use without requiring to be directly within a liquid by any operation by the user, that the lubricious characteristic of the hydrophilic surface is not negatively affected although it is dipped in water, that has a hydrophilic surface that does not lose its properties for an extended period of time when it is adhered to a catheter manufactured from PVC-free materials.

### Brief Description of the Invention

A purpose of this invention is to provide a catheter set comprising a catheter wherein a hydrophilic surface is directly placed in water and does not require an extra water compartment.

Another purpose of this invention is to provide a catheter set wherein the hydrophilic surface placed in water does not lose its lubricious characteristics for an extended period of time.

Another purpose of this invention is to provide a catheter set wherein the hydrophilic surface properties are maintained for an extended period of time even when the catheter used is manufactured from PVC-free material.

Within the scope of this invention a catheter which is manufactured from medical grade PVC or Thermoplastic Elastomer-based PVC-free materials, is used as a support material. In this invention PVP (Polyvinylpyrrolidone) coating material is used as a hydrophilicity and lubrication enhancer, Methylene chloride is used a solvent, Erucamide is used as a lubrication and stability enhancer, Toluene diisocyanate (TDI) is used as a crosslinker, Sodium chloride is used as a glidant, Chitosan is used as an antibacterial agent and lubrication enhancer, Polyvinyl alcohol (PVA) is used as a lubrication enhancer, Polyethylene oxide (PEO) is used as a lubrication enhancer, Sorbic acid is used as a lubrication enhancer, Acrylic acid is used as a solvent, Hydroxyethyl cellulose (HES) is used as a lubrication enhancer, Titanium dioxide is used as a lubrication enhancer, Toluene is used as a solvent and surface cleaner, Benzoyl peroxide is used as an initiator and pure water is used as a solvent.

Surfaces that have been coated with PVP (Polyvinylpyrrolidone) using the dipping method have been bonded with a antibacterial agent and a lubrication and water retention capacity enhancer again using the dipping method, and thereby a catheter manufactured from PVC or PVC-free materials that is antibacterial, hydrophilic, highly lubricated thus with a low friction coefficient and the adhesion between the hydrophilic coating and the catheter surface is absolute and has a dense surface that can preserve its lubrication in water for an extended period of time, has been provided.

### Detailed Description of the Invention

The catheter set of the invention is shown in the enclosed drawings.

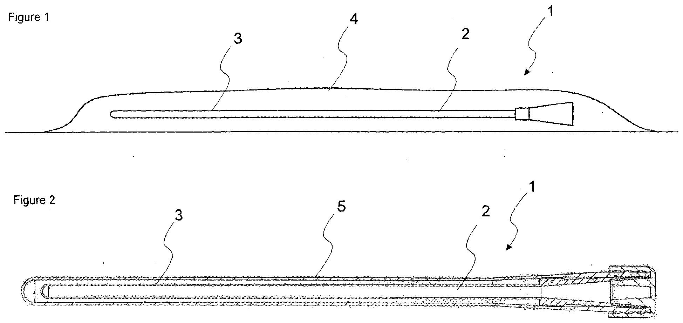

FIGURE 1- is a schematic view of an embodiment of the catheter set.
FIGURE 2 - is a schematic view of another embodiment of the catheter set.

The parts in the drawings are individually numbered and are identified below.
1. Catheter set
2. Catheter
3. Hydrophilic surface
4. Bag shaped catheter package
5. Tube shaped catheter package

The coating of the catheter (2) surface with an antibacterial, hydrophilic solution that is lubricated with water and preserves this lubrication over an extended period of time, is performed in 3 steps. Three different solutions are prepared and these solutions are applied to the catheter (2) separately.

### Solution No.1

Solution No.1 is comprised of 97-99% toluene, 1-3% methylene chloride and 0.1-0.2% benzoyl peroxide, by weight. To attain solution no.1 while Toluene and methylene chloride are being stirred Benzoyl peroxide is added to the mixture and stirred to enable the benzoyl peroxide to dissolve.

### Solution No.2

Solution No.2 is comprised of two separate solutions defined as A and B being mixed together. The A solution is 50-55% and the B solution is 45-50% by weight within the overall solution. Herein the solution A is comprised of 85-92% methylene chloride, 2-5% toluene, 0.1-0.3% erucamide and 5-10% PVP by weight. Solution B is comprised of 94-97% methylene chloride, 1-3% toluene and 1-3% TDI by weight. Solution 2 is obtained by mixing solution A and solution B.

For the preparation of Solution A, erucamide is dissolved by being stirred in methylene chloride. After the Erucamide is dissolved in full toluene is added and stirred. Then the PVP is added and stirred and PVP is totally dissolved. Solution B is prepared separately. For this methylene chloride and toluene are stirred and TDI is added and stirred. Finally the solution stated as A is added into the solution stated as B while it is being stirred and the overall solution is stirred to obtain Solution 2.

### Solution No.3

Solution No.3 is comprised of a mixture of six different solutions defined as a, b, c, d, e and f. Herein the a, b and c solutions are used as a base and all or a selection of the d, e, and f solutions are added to the coating solution. The solution no.3 is comprised of a mixture of 30-50% a, 30-50% b, 10-20% c, 3-5% d, 10-20% e and 0.5-1.5% f by weight.

The solution a is comprised of 90-96% pure water, 1-2% acrylic acid, 1-4% Chitosan and 1-4% polyvinyl alcohol by weight.

The solution b is comprised of 77-87% pure water, 1-3% PVP and 10-20% sodium chloride by weight.

The solution c is comprised of 2-4% polyethylene oxide and 96-98% pure water by weight.

The solution d is comprised of 1-3% HES and 97-99% pure water by weight.

The solution e is comprised of 0.1-0.3% sorbic acid and 99.7-99.9% pure water by weight.

The solution f is comprised of 1-1.5% TiO₂ and 98.5-99% pure water by weight.

To prepare the solution a, acrylic acid is added to pure water and stirred. Chitosan is added along with polyvinyl alcohol and stirring is continued. Chitosan and PVA are totally dissolved by stirring the solution.

To prepare the solution b, PVP and sodium chloride is dissolved in pure water by stirring.

To prepare the solution c, polyethylene oxide is dissolved in pure water by stirring.

To prepare the solution d, HES is dissolved in pure water by stirring.

To prepare the solution e, sorbic acid is dissolved in pure water by stirring.

To prepare the solution f, TiO₂ is stirred in pure water.

The prepared solutions of a, b, c, d, e and f are mixed and stirred with one or more of the solutions d, e and f with the solutions of a, b and c used as a base. As a result of this stirring solution No.3 is prepared.

After the preparation of solution I, II and III, the process of coating of at least one section of the surface is performed. The nelaton (uncoated) catheters (2) are put on stainless steel skewers one by one and then they are dipped into the pre-prepared coating solutions and dried in a furnace after coating the catheter (2) surfaces with gel, their urinary holes are drilled with a drilling machine and then the catheters (2) are placed one by one inside packages (4 or 5) filled with pure water and the lids of the packages are tightly closed and they are sterilized with gamma radiation sterilization. For the coating process the steps below are followed.
- The catheters (2) which put on stainless steel skewers are dipped into the solution I, they are kept inside this solution for 20-60 seconds and then they are taken out. The dipped catheters (2) are dried in a furnace at 50°C and then they are allowed to cool down.
- After allowed to cool down, the catheters which are subjected to the process of solution I are dipped into the solution II and then dried in a furnace at 50°C for 20 minutes. Later, they are taken out of the furnace and allowed to cool down.

- The catheters (2) which are dipped into the solution II and which are allowed to cool down, are then dipped into the solution III, kept inside this solution for 10-40 seconds and then they are taken out of the solution and dried at 70-90°C and after the drying process they are allowed to cool down.
- Their urinary holes are drilled with a drilling machine.
- The catheters (2) are placed one by one inside the catheter packages (4 or 5) which are filled with pure water and the lids of the packages are tightly closed.
- The catheters (2) which are placed inside the catheter packages (4 or 5) filled with pure water are then sterilized with gamma radiation sterilization.
- The catheter packages (4 or 5) can be in the form of a plastic bag (4) impermeable to water and air or they can have tube shapes (5).

With the coating which can be applied on surfaces of the PVC or PVC-free TPE based nelaton catheters (2) by method of dipping into the three solutions, a catheter set (1) is obtained which has a dense surface which swells up when contacted with water due to its hydrophilic characteristic, which is extremely lubricious, which maintains its lubricious characteristic inside water for a long time and wherein the adherence between the hydrophilic surface (3) and the catheter (2) is complete and the drying period is long, which does not adhere not possess any roughness, which has a blurry white color and antibacterial characteristics due to its chitosan and PVP contents.

By means of the catheter set (1), which is the subject of the invention and by placing the catheters inside plastic tubes (5) or bags (4) filled with pure water provides ease of use for the patient. There is no need for an extra external water chamber so the patient does not have to perform an additional process such as having to contact the water with the catheter (2).

Placing the catheter (2) inside a catheter package (4 or 5) filled with pure water enables homogenous wetting of the hydrophilic surface (3) which is located on the catheter (2) surface and in this way the hydrophilic surface (3) located on the catheter (2) surface is water-logged and its lubricious characteristic is increased in terms of usage.

Placing the catheters (2) inside plastic tubes (5) filled with pure water which can be easily opened again provides ease of use. In this way, the uneasiness of the patient when opening the package (5) and spillage of the water inside the package (5) is eliminated.

## Claims

1. A production method of a ready to use catheter set (I) with the steps of;
- obtaining solution I hy adding and mixing to the mixture % 0,1-0,2 benzoyl peroxide while mixing % 97-99 toluene and % 1-3 methylene chloride, hy weight,
- obtaining solution II by mixing solution A comprising of % 85-92 methylene chloride, % 2-5 toluene, % 0,1-0,3 crucamide and % 5-10 PVP by weight, and solution B comprising of % 94-97 methylene chloride, 1-3 toluene, % 1-3 TDI, by weight,
- obtaining solution III by mixing one or more of solution a comprising of % 90-96 pure water, % 1-2 acrylic acid, % 1-4 Chitosan and % 1-4 polyvinyl alcohol by weight, solution b comprising of % 77-87 pure water, % 1-3 PVP and% 10-20 sodium chloride by weight, solution c comprising of % 2-4 polyethylene oxide and % 96-98 pure water by weight, solution d comprising of % 1-3 HES and % 97-99 pure water by weight, solution e comprising of % 0. 1-0,3 sorbic acid and % 99,7-99,9 pure water by weight and solution f comprising of % 1-1,5 TiO₂ and % 98,5-99 pure water by weight.
- dipping the catheters (2) put on the skewers into the solution I, keeping them inside this solution for 20-60 seconds and then taking them out, later drying them in a furnace at 50°C and allowing them to cool down,
- after they arc cooled down dipping them inside solution II and drying them in a furnace at 50°C for approximately 20 minutes and then allowing them to cool down,
- after they are cooled down dipping them into the solution III, keeping them inside this solution for 10-40. seconds and then taking them out and drying them in a furnace at 70-90°C and then allowing them to cool down,
- Packaging the catheters (2) by placing them one by one inside catheter packages (4 or 5) filled with pure water.

2. A production method of a catheter set (1) according to claim 1 **characterized in that** solution II comprises % 50-55 solution A and % 45-50 solution B by weight.

3. A. production method of a catheter set (1) according to claim 2 **characterized in that** solution III comprises % 30-50 solution a, % 30-50 solution h, % 10-20 solution c, % 3-5 solution d, % 10-20 solution e and % 0,5-1,5 solution f by weight.

## Patentansprüche

1. Eine Produktionsmethode für einen gebrauchsfertigen Katheterapparat (1) mit den Schritten von;
- Erhaltung der Lösung I durch Hinzufügung und Einmischung zur Mischung 0,1-0,2% Benzoylperoxid, wobei Einmischung von 97-99% Toluen und 1-3% Methylenchlorid, nach Gewicht,
- Erhaltung der Lösung II durch Einmischung von Lösung A bestehend aus 85-92% Methylenchlorid, 2-5% Toluen, 0,1-0,3% Erucamid und 5-10% PVP nach Gewicht, und von Lösung B bestehend aus 94-97% Methylenchlorid, 1-3% Toluen, 1-3% TDI, nach Gewicht,
- Erhaltung der Lösung III durch Einmischung von einer oder mehreren Lösungen wie Lösung a bestehend aus 90-96% Reinwasser, 1-2% Acrylsäure, 1-4% Chitosan und 1-4% Polyvinylalkohol nach Gewicht, Lösung b bestehend aus 77-87% Reinwasser, 1-3% PVP und 10-20% Natriumchlorid nach Gewicht, Lösung c bestehend aus 2-4% Polyethylenoxid und 96-98% Reinwasser nach Gewicht, Lösung d bestehend aus 1-3% HES und 97-99% Reinwasser nach Gewicht, Lösung e bestehend aus 0,1-0,3% Sorbinsäure und 99,7-99,9% Reinwasser nach Gewicht und Lösung f bestehend aus 1-1,5% TiO₂ und 98,5-99% Reinwasser nach Gewicht,
- Eintauchung der Kathetern (2), die auf Spieße in Lösung I hingelegt werden, deren Behalten in diese Lösung für 20-60 Sekunden und dann deren Herausnahme, danach deren Trocknung in einem Hochofen bei 50°C und deren Abkühlung,
- Nach deren Abkühlung, deren Eintauchung in die Lösung II und deren Trocknung in einem Hochofen bei 50°C für nahezu 20 Minuten und dann deren Abkühlung,
- Nach deren Abkühlung, deren Eintauchung in die Lösung III, deren Behalten in dieser Lösung für 10-40 Sekunden und danach deren Herausnahme und Trocknung in einem Hochofen bei 70-90°C und dann deren Abkühlung,
- Einpackung der Kathetern (2) durch deren Einlegung eine nach dem anderen in Kathetereinpackungen (4 oder 5) gefüllt mit Reinwasser.

2. Eine Produktionsmethode von einem Katheterapparat (1) nach Anspruch 1 **gekennzeichnet dadurch, daß** Lösung II beinhaltet 50-55% von Lösung A und 45-50% von Lösung B nach Gewicht.

3. Eine Produktionsmethode von einem Katheterapparat (1) nach Anspruch 2 **gekennzeichnet dadurch, daß** Lösung III beinhaltet 30-50% von Lösung a, 30-50% von Lösung b, 10-20% von Lösung c, 3-5% von Lösung d, 10-20% von Lösung e und 0,5-1,5% von Lösung f nach Gewicht.

## Revendications

1. Procédé de production d'un ensemble de cathéter (1) prêt à l'emploi avec les étapes suivantes;
- obtention de la solution I par addition et mélangant au mélange de peroxyde de benzoyle 0,1-0,2% tout en mélangeant 97-99% toluène et 1-3% chlorure de méthylène, en poids,
- obtention de la solution II par mélange de la solution A comprenant du chlorure de méthylène 85-92%, du toluène 2-5%, du 0,1-0,3% érucamide et du 5-10% PVP en poids, et de la solution B constituée de 94-97% méthylène chlorure, 1-3% toluène, 1-3% TDI, en poids,
- obtention de la solution III en mélangeant une ou plusieurs des solutions comprenant de 90-96% l'eau pure, 1-2% acide acrylique, 1-4% Chitosan et 1-4% alcool polyvinylique en poids, solution b comprenant 77-87% l'eau pure, 1-3% PVP et 10-20% chlorure de sodium en poids, la solution c comprenant de l'oxyde de polyéthylène 2-4% et de l'eau pure 96-98% en poids, solution d comprenant de l'eau pure à 1 à 3% HES en poids et de l'eau pure à 97-99% en poids, la solution e comprenant de l'acide sorbique en 0,1-0,3% et de l'eau pure en 99,7-99,9% en poids et une solution f comprenant de l'eau pure en 1-1,5% TiO₂ et 98,5-99% en poids,
- tremper les cathéters (2) mis sur les brochettes dans la solution I, les garder à l'intérieur de cette solution pendant 20-60 secondes, puis en les sortant, plus tard, les sécher dans un four à 50°C et leur permettre de refroidir,
- après les avoir refroidis, les tremper dans la solution II et les sécher dans un four à 50°C pendant environ 20 minutes, puis les laisser refroidir,
- après les avoir refroidis, les tremper dans la solution III en les gardant à l'intérieur de cette solution pendant 10 à 40 secondes, puis les retirer et les sécher dans un four à 70-90°C, puis les laisser refroidir,
- Conditionner les cathéters (2) en les plaçant un à un dans des boîtiers de cathéter (4 ou 5) remplis d'eau pure.

2. Procédé de production d'un ensemble de cathéter (1) selon la revendication 1, **caractérisé en ce que** la solution II comprend 50-55% solution A et 45-50% solution B en poids.

3. Procédé de fabrication d'un ensemble de cathéter (1) selon la revendication 2, **caractérisé en ce que** la solution III comprend 30-50% solution a, 30-50% solution b,% 10-20 solution c, 3-5% solution d, 10-20% solution e et 0,5-1,5% solution f en poids.
